(19) Europäisches Patentamt
European Patent Office
Office européen des brevets

(11) Veröffentlichungsnummer: **0 267 488**
**B1**

(12) # EUROPÄISCHE PATENTSCHRIFT

(45) Veröffentlichungstag der Patentschrift: **09.05.90**

(51) Int. Cl.⁵: **C07C 255/23**, C07C 253/00

(21) Anmeldenummer: **87115754.1**

(22) Anmeldetag: **27.10.87**

(54) **Verfahren zur Herstellung monomerer Alpha-Cyanacrylate.**

(30) Priorität: **08.11.86 DE 3638171**

(43) Veröffentlichungstag der Anmeldung:
**18.05.88 Patentblatt 88/20**

(45) Bekanntmachung des Hinweises auf die Patenterteilung:
**09.05.90 Patentblatt 90/19**

(84) Benannte Vertragsstaaten:
**DE FR GB IT**

(56) Entgegenhaltungen:
**DE-A- 1 568 479**

(73) Patentinhaber: **BAYER AG,**
**D-5090 Leverkusen 1 Bayerwerk(DE)**

(72) Erfinder: **Arlt, Wolfgang, Dr., Schönwasserstrasse 230 c,**
**D-4150 Krefeld(DE)**
Erfinder: **Waniczek, Helmut, Dr., Wolfskaul 2,**
**D-5000 Köln 80(DE)**
Erfinder: **Viard, Richard, Dr.,**
**Friedrich-Weskott-Strasse 6, D-5090 Leverkusen(DE)**
Erfinder: **Brück, Dieter, Dr., Kattowitzer Strasse 34,**
**D-5000 Köln 80(DE)**

**EP 0 267 488 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein Verfahren zur Herstellung monomerer α-Cyanacrylate durch pyrolytische Spaltung von Poly-α-cyanacrylaten und anschließende Destillation der erhaltenen monomeren α-Cyanacrylate.

Es ist bekannt, daß monomere α-Cyanacrylate sehr leicht polymerisieren, wenn sie Verunreinigungen enthalten. Diese Instabilität bereitet bei der Herstellung Schwierigkeiten und vermindert die Ausbeuten. Außerdem sind monomere α-Cyanacrylate nur sehr begrenzt lagerstabil, wenn sie auch nur geringe Mengen Veruneinigungen enthalten, was dann ihre Verwendbarkeit, z. B. als Klebstoffe, beeinträchtigt. Es ist daher erforderlich, die monomeren α-Cyanacrylate, die bei der Pyrolyse von Poly-α-cyanacrylaten entstehen, zu reinigen, z. B. durch Destillation.

Die bekannten Destillationsverfahren zur Reinigung der monomeren α-Cyanacrylate sind aufwendig und beeinträchtigen die Ausbeuten. Weiterhin sind bislang nur Destillationsverfahren bekannt, die höchstens fünf Trennnstufen in der Destillationskolonne haben (z.B. DE-OS 2 231 561). Mit derartigen Destillationsverfahren können nur dann hochreine α-Cyanacrylate erhalten werden, wenn diese Destillationsverfahren mehrmals durchgeführt werden. Das hat eine Verminderung der Ausbeute an monomerem α-Cyanacrylat zur Folge.

Es wurde gefunden, daß bereits in einem einzigen Reinigungsschritt monomere α-Cyanacrylate rein erhalten werden können, wenn sie in Gegenwart von Polymerisationsinhibitoren im Gegenstrom über mehr als zehn (10) Trennstufen destilliert werden.

Gegenstand der Erfindung ist ein Verfahren zur Herstellung hochreiner, monomerer α-Cyanacrylate durch Destillation bei vermindertem Druck, dadurch gekennzeichnet, daß im Gegenstrom mit mehr als 10 Trennstufen bei vermindertem Druck destilliert wird und auf der obersten Trennstufe dem Gegenstrom kontinuierlich Inhibitoren zugeführt werden und an den Stellen, an denen Kondensat auftreten kann, stabilisiertes flüssiges α-Cyanacrylat zugeführt wird.

Erfindungsgemäß kann der Gegenstrom in einem Gegenstromapparat mit Dephlegmator erzeugt werden.

Bei der Durchführung der Erfindung wird der Abstand der einzelnen Trennstufen so gewählt, daß die nächstobere Trennstufe stets im Spritzbereich der unteren liegt. Der Dephlegmator oder Kühler ragt in den Spritzbereich des obersten Bodens. An den Stellen des Gegenstromapparates, an denen aufgrund der niedrigen α-Cyanacrylatkonzentration keine Polymerisationsgefahr besteht, ist es nicht erforderlich stabilisierte Flüssigkeit an das Kondensat heranzuführen, kann aber ebenfalls vorteilhaft sein.

Bei der Durchführung des erfindungsgemäßen Verfahrens wird der Gegenstrom im Gegenstromapparat mit einem Dephlegmator erzeugt, dessen Betriebstemperatur so gewählt wird, daß tiefer als das α-Cyanacrylat siedende Verunreinigungen dampfförmig passieren können, während das α-Cyanacrylat kondensiert wird. Die Temperatur des Dephlegmators wird so gewählt, daß sie unterhalb der Siedetemperatur des α-Cyanacrylates, aber mehr als 30°C, bevorzugt mehr als 50°C, oberhalb der Siedetemperatur des dem Cyanacrylat zugrundeliegenden Alkohols beim jeweiligen Destillationsdruck liegt.

Unter α-Cyanacrylat werden Monomere der Formel I

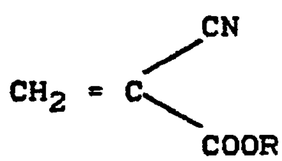

(I),

verstanden, wobei R ein verzweigter oder unverzweigter, substituierter oder unsubstituierter Alkyl- oder Cycloalkylrest mit 1 bis 10 C-Atomen ist.

Der erfindungsgemäß zu verwendende Gegenstromapparat ist beispielsweise eine Bodenkolonne mit 10 bis 50 Trennstufen. Bevorzugt wird eine Bodenkolonne mit 20 bis 50 Trennstufen eingesetzt. Der Gegenstromapparat wird bei vermindertem Druck betrieben, vorzugsweise bei einem Druck von 5 bis 100 mbar, besonders bevorzugt bei einem Druck von 10 bis 50 mbar.

Erfindungsgemäß wird der Rücklauf an einem der obersten Böden in zwei Teile aufgeteilt, wobei der eine Teil als Produktstrom entnommen wird und der andere Teil zur Erzeugung des Gegenstroms auf den nächst unteren Boden geführt wird.

Der erfindungsgemäß zu verwendende Gegenstromapparat besitzt auf der obersten Trennstufe eine Öffnung mit der Möglichkeit, einen oder mehrere Inhibitoren, getrennt oder gemeinsam, kontinuierlich einzudosieren.

Die verwendeten Inhibitoren verhindern eine anionische und eine radikalische Polymerisation. In der Regel werden mindestens ein Inhibitor, gegen die anionische und ein Inhibitor gegen die radikalische Polymerisation getrennt oder gemeinsam, in Substanz oder gelöst eindosiert.

Es kann vorteilhaft sein, zusätzlich gasförmige Inhibitoren wie $SO_2$ während der Destillation durch den erfindungsgemäß verwendeten Gegenstromapparat zu leiten.

Erfindungsgemäß können nur Inhibitoren gegen die radikalische Polymerisation verwendet werden, die eine radikalische Polymerisation des Cyanacrylats verhindern, aber nicht die anionische Polymerisation starten. Beispielsweise können Hydrochinon, Brenzkatechin, Resorcin oder deren Derivate wie Hydrochinon-monomethylether oder Phenole wie Di-tert.-butylphenol oder 2,6-Di-tert.-butyl-p-kresol, 2,2'-Methylen-bis-(4-methyl-6-tert.butylphenol), Bisphenol A, Dihydroxydiphenylmethan, styrolisierte Phenole verwendet werden.

Die Inhibitoren gegen die anionische Polymerisation die erfindungsgemäß verwendet werden können, drängen die anionische Polymerisation der Cyanacrylate zurück. Beispielsweise können starke Säuren, Lewis-Säuren, Schwefelsäure, Salzsäure, Sulfonsäuren wie Methan-, Ethan- oder höhere Sulfonsäuren, p-Toluolsulfonsäure, Phosphorsäure oder Polyphosphorsäuren, Silylester starker Säuren wie Trialkylchlorsilane, Dialkyldichlorsilane, Alkyltrichlorsilane, Tetrachlorsilan, Trialkylsilylsulfonsäuren, Trialkylsilyl-p-toluolsulfonate, Bistrialkylsilylsulfat und Trialkylsilylphosphorsäureester verwendet werden.

Die Inhibitoren gegen die radikalische Polymerisation werden auf den obersten Boden im erfindungsgemäß verwendeten Gegenstromapparat in einer solchen Menge zugegeben, daß das von Dephlegmator rücklaufende Kondensat, welches dann den Gegenstrom bildet, 0,005 bis 2 Gew.-% Inhibitor enthält. Bevorzugt wird ein Gehalt von 0,01 bis 1 Gew.-% Inhibitor zugegeben.

Die Inhibitoren gegen eine radikalische oder gegen eine anionische Polymerisation können einzeln oder als Mischung gleichzeitig oder nacheinander zugegeben werden.

So wird als Destillationsprodukt bereits ein stabilisiertes α-Cyanacrylat erhalten. Es ist daher ein weiterer Vorteil des erfindungsgemäßen Verfahrens, daß so die problematische Handhabung nicht stabilisierten α-Cyanacrylats entfällt und auf eine weitere Zugabe von Stabilisatoren verzichtet werden kann.

Die Inhibitoren gegen die anionische Polymerisation werden erfindungsgemäß am obersten Boden des Gegenstromapparates in einer solchen Menge zugegeben, daß der Gegenstrom 1 ppm bis 1000 ppm Inhibitor enthält. Bevorzugt wird ein Gehalt von 5 ppm bis 500 ppm, ganz besonders bevorzugt 10 ppm bis 100 ppm Inhibitor im Gegenstrom.

Da die erfindungsgemäß einzusetzenden Inhibitoren Feststoffe sein können, kann es vorteilhaft sein, diese in verdünnten Lösungen einzusetzen. Als Lösungsmittel kommen inerte Lösungsmittel in Frage, beispielsweise verzweigte oder unverzweigte, aliphatische, cyclische oder alicyclische, aromatische oder araliphatische, substituierte oder unsubstituierte Kohlenwasserstoffe wie n-Hexan, n-Heptan, n-Octan, Cyclopentan, Cyclohexan, 2,2,4-Trimethylpentan, Benzol, Toluol, Chloroform oder Tetrachlorkohlenstoff oder Cyanacrylate. Es kann von Vorteil sein, wenn die verwendeten Lösungsmittel Azeotrope mit Wasser oder Alkoholen bilden können.

Der erfindungsgemäß verwendete Gegenstromapparat (s. Fig. 1) ist so konstruiert, daß an die Stellen, an denen Kondensat auftreten kann, stabilisiertes α-Cyanacrylat hingeführt wird. Dies kann durch geeigneten Abstand der Trennstufen erreicht werden. Der Abstand zwischen zwei Trennstufen ist kürzer als der Spritzbereich der jeweils unteren Trennstufe. Der Dephlegmator ragt in den Spritzbereich der obersten Trennstufe hinein.

Der Dephlegmator ist eine Vorrichtung oberhalb der obersten Trennstufe. Im Dephlegmator wird ein Teil der im erfindungsgemäßen Gegenstromapparat aufsteigenden Dämpfe kondensiert, woduch ein Gegenstrom erzeugt wird. Der Dephlegmator wird bei einer Temperatur betrieben, so daß leichter als Cyanacrylat siedende Verunreinigungen dampfförmig passieren können, während das Cyanacrylat kondensiert wird.

Die Betriebstemperatur ($T_{Deph}$) des Dephlegmators wird in Abhängigkeit vom jeweiligen Destillationsdruck von 5 bis 100 mbar gewählt. Diese Temperatur wird so gewählt, daß sie unterhalb der Siedetemperatur ($T_1$) des α-Cyanacrylsäureesters beim angewendeten Destillationsdruck, aber oberhalb der Siedetemperatur ($T_2$) des dem α-Cyanacrylsäureester zugrundeliegenden Alkohols beim jeweilig angewendeten Destillationsdruck liegt. Die Dephlegmatortemperatur ($T_{Deph}$) beträgt:

$(T_2) < (T_{Deph.}) < (T_1)$.

Bevorzugt beträgt ($T_{Deph}$)

$(T_2+30°C) < (T_{Deph}) < (T_1-10°C)$,

besonders bevorzugt beträgt ($T_{Deph}$)

$(T_2+40°C) < (T_{Deph}) < (T_1-25°C)$.

Bei dem erfindungsgemäßen Verfahren wird das hochreine α-Cyanacrylat als flüssige Phase von einem der oberen Böden des Gegenstromapparates im Seitenstrom abgezogen. Bevorzugt wird das α-Cyanacrylat von einem der obersten acht Böden abgezogen.

Die erfindungsgemäß hergestellten hochreinen α-Cyanacrylagte eignen sich als schnell abbindende, lagerstabile Klebstoffe. Zur Einstellung bestimmter Eigenschaften wie Viskosität, Farbe oder Abbindegeschwindigkeit können sie mit üblichen Zusätzen, Polymeren, Farbstoffen oder Stabilisatoren versetzt werden.

Bei der Durchführung des erfindungsgemäßen Destillationsverfahren kann die in Figur 1 und 2 erläuterte Destillationsapparatur verwendet werden.

Figur 1 zeigt eine Glockenbodenkolonne der Verstärkersäule, in welcher bedeutet:

1 = Kühlmantel des Dephlegmators (liegt im Spritzbereich der Glocke 6),

2 = Ablaufschacht,

3 = oberste Glocke,

4 = Unterseite des Glockenbodens (liegt im Spritzbereich der Glocke 7),
5 = Spritzbereich der jeweiligen Glocken,
6,7,8,9 = Glocken.

Eine mögliche Anordnung zur technischen Durchführung ist in Fig. 2 wiedergegeben: 11 ist die Verstärker- und 12 ist die Abtreibersäule einer Destillationskolonne. In diese Kolonne fließt mit Strom 10 das zu trennende Stoffgemisch. Strom 18 sind die zur Durchführung des erfindungsgemäßen Verfahrens notwendigen Inhibitoren, die mit dem aus dem Dephlegmator 13 kommenden Flüssigkeitsstrom vereinigt werden. Die nicht im Dephlegmator kondensierten Dämpfe, insbesondere der gasförmige Inhibitor, oder leichtsiedende Komponenten, werden in einem tiefgekühlten Kondensator 14 verflüssigt und verworfen. Der Kondensator ist über Ausgang 19 mit einer Vakuumanlage verbunden.

Der zum Betrieb der Destillationskolonne notwendige Produktdampf wird im Verdampfer 15 erzeugt, der durch seine Bauweise durch geringe Temperaturbelastung und Verweilzeit eine schonende Verdampfung des Sumpfproduktes erlaubt. Das Sumpfprodukt verläßt mit Strom 17 die Destillationskolonne. Mit Strom 20 können gasförmige Inhibitoren in die Kolonne eingeführt werden.

Strom 16 ist der einige Trennstufen unterhalb des Dephlegmators gelegene Reinprodukt-Ausgang. Dabei wird geregelt nur eine solche Menge Reinprodukt flüssig entnommen, daß der in der Verstärkersäule abwärts fließende Strom zur Stofftrennung gerade ausreicht.

<u>Beispiele</u>

Eine nach dem Prinzip der Fig. 1 und 2 aufgebaute Labordestillationsanlage hatte 20 Glockenböden in der Abtreibersäule und 23 Glockenböden ohne Zwischenboden in der Verstärkersäule. Der Produktstrom 16 wurde auf dem dritten Boden unterhalb des Dephlegmators flüssig abgenommen. Die Abnahmemenge war so eingestellt, daß das Verhältnis zwischen entnommener Menge und in der Verstärkersäule weiterfließender Menge 1:4 betrug. Der Dephlegmator wurde bei 45°C betrieben und war im Spritzbereich des obersten Bodens. Der Sumpfverdampfer 15 war ein Fallfilmverdampfer. Die Destillationsanlage wurde bei 20 mbar Kopfdruck betrieben. Es wurde folgende Stoffströme eingestellt bzw. erreicht:

<u>Strom 10</u> (Zulauf): 273 g/h

86,1% Cyanacrylsäureethylester (technische Qualität)
13,9% Nebenprodukt

<u>Strom 18</u> (Inhibitor-Zulauf): 19,6 g/h

0,03% Bis-trimethylsilylsulfat
1,0% Vulkanox-BKF® Bayer AG (= 2,2'-Methylen-bis-(4-methyl-6-tert.-butylphenol))
in n-Hexan

<u>Strom 16</u> (Reinprodukt-Entnahme): 219,2 g/h

> 99,5% Cyanacrylsäureethylester
< 0,5% sonstige Produkte

<u>Strom 17</u> (Sumpfentnahme): 29,9 g/h

100 % schwersiedende Nebenkomponenten

In dem Tiefkühler 14 wurden nach einer Stunde Destillation 44 g Leichtsieder abdestilliert. Auch nach einer mehrwöchigen Destillation war keine Polymerbildung zu erkennen.

<u>Definitionen verwendeter Begriffe</u>

Trennstufe

Eine Trennstufe führt in einem Gegenstromapparat zu einem Massen- und Energieaustausch und damit zu einer Veränderung der Ströme. Eine Trennstufe entspricht in der Destillationstechnik dem Boden einer Bodenkolonne oder einer bestimmten Packungshöhe in einer Kolonne mit Füllkörpern.

Kondensat

Unter Kondensat wird anmeldungsgemäß die aus der Dampfphase kondensierende Flüssigkeit (z. B. an kälteren Wänden, an dem nächstoberen Boden) verstanden, die aufgrund ihres Gehaltes an Cyanacrylat und dem Fehlen der flüssigen Inhibitoren zur Polymerisation neigt.

Rücklaufteiler

In einer Destillationskolonne als Gegenstromapparat fließt ein Flüssigkeitsstrom von oben nach unten und ein Dampfstrom von unten nach oben. Der Dampfstrom wird im wesentlichen durch die unten zugeführte Wärme bestimmt, der Flüssigkeitsstrom im wesentlichen durch die Aufteilung des Kondensats des Kondensators bzw. des Dephlegmators in den Produktstrom und den Rücklaufstrom. Diese Aufgabe übernimmt der Rücklaufteiler.

## Patentansprüche

1) Verfahren zur Herstellung hochreiner, monomerer α-Cyanacrylate durch Destillation bei vermindertem Druck, dadurch gekennzeichnet, daß im Gegenstrom und mit mehr als 10 Trennstufen bei vermindertem Druck destilliert wird und auf der obersten Trennstufe dem Gegenstrom kontinuierlich Inhibitoren zugeführt werden und an den Stellen, an denen Kondensat auftreten kann, stabilisiertes flüssiges α-Cyanacrylat zugeführt wird.

2) Verfahren nach Anspruch 1, dadurch gekennzeichnet, daß flüssiges, stabilisiertes α-Cyanacrylat in einem der obersten 8 Böden, vom Dephlegmator aus gezählt, entnommen wird.

3. Verfahren nach Anspruch 1, dadurch gekennzeichnet, daß die Dephlegmatortemperatur ($T_{Deph}$) unterhalb der Siedetemperatur ($T_1$) des α-Cyanacrylsäureesters beim angewendeten Destillationsdruck von 5 bis 100 mbar, aber oberhalb der Siedetemperatur ($T_2$) des dem α-Cyanacrylsäureester zugrundeliegenden Alkohols beim jeweilig angewendeten Destillationsdruck

$(T_2) < (T_{Deph}) < (T_1)$

beträgt.

4) Verfahren nach Anspruch 1, dadurch gekennzeichnet, daß das im Dephlegmator erzeugte Kondensat mit 0,005 bis 2 Gew.% Inhibitoren gegen die radikalische Polymerisation und 1 bis 1000 ppm Inhibitoren gegen die anionische Polymerisation stabilisiert wird.

5) Verfahren gemäß Anspruch 1, dadurch gekennzeichnet, daß der Dephlegmator bis in den Spritzbereich der obersten Trennstufe reicht.

6) Verfahren gemäß Anspruch 1, dadurch gekennzeichnet, daß der Abstand der Trennstufen so gewählt wird, daß die nächst obere Trennstufe im Spritzbereich der unteren liegt.

## Claims

1. A process for the production of high-purity, monomeric α-cyanoacrylates by distillation under reduced pressure, characterized in that distillation is carried out in countercurrent under reduced pressure and with more than 10 separation stages, inhibitors are continuously added to the countercurrent at the uppermost separation stage and stabilized liquid α-cyanoacrylate is added at those places where condensate can occur.

2. A process as claimed in claim 1, characterized in that liquid, stabilized α-cyanoacrylate is removed from one of the uppermost 8 plates, counting from the dephlegmator.

3. A process as claimed in claim 1, characterized in that the dephlegmator temperature ($T_{deph}$) is below the boiling temperature ($T_1$) of the α-cyanoacrylate under the distillation pressure of 5 to 100 mbar applied, but above the boiling temperature ($T_2$) of the alcohol on which the α-cyanoacrylate is based at the particular distillation pressure applied

$(T_2) < (T_{deph}) < (T_1)$.

4. A process as claimed in claim 1, characterized in that the condensate produced in the dephlegmator is stabilized against radical polymerization with 0.005 to 2% by weight inhibitors and against anionic polymerization with 1 to 1000 ppm inhibitors.

5. A process as claimed in claim 1, characterized in that the dephlegmator extends into the spray range of the uppermost separation stage.

6. A process as claimed in claim 1, characterized in that the interval between the separation stages is selected so that the next upper separation stage lies in the spray range of the lower separation stage.

## Revendications

1. Procédé de production d'α-cyanacrylates monomères très purs par distillation sous pression réduite, caractérisé en ce que la distillation est coduite à contre-courant et avec plus de dix étages de séparation sous pression réduite et en ce que des inhibiteurs entrent continuellement en contact avec le contre-courant à l'étage de séparation le plus haut, et de l'α-cyanacrylate liquide stabilisé est amené aux endroits où un condensat peut apparaître.

2. Procédé suivant la revendication 1, caractérisé en ce que l'α-cyanacrylate stabilisé liquide est déchargé dans l'un des huit plateaux les plus hauts, comptés à partir du déphlegmateur.

3. Procédé suivant la revendication 1, caractérisé en ce que la température ($T_{Deph}$) du déphlegmateur est inférieure à la température d'ébullition ($T_1$) de l'ester d'acide α-cyanacrylique à la pression de distillation utilisée de 5 à 100 mbars, mais au-dessus de la température d'ébullition ($T_2$) de l'alcool à la base de l'ester d'acide α-cyanacrylique à la pression de distillation utilisée dans chaque cas
($T_2$) < ($T_{Deph}$) < ($T_1$).

4. Procédé suivant la revendication 1, caractérisé en ce que le condensat produit dans le déphlegmateur est stabilisé contre la polymérisation radicalaire avec 0,005 à 2% en poids d'inhibiteurs et contre la polymérisation anionique avec 1 à 1000 ppm d'inhibiteurs.

5. Procédé suivant la revendication 1, caractérisé en ce que le déphlegmateur s'étend jusque dans la zone d'injection de l'étage de séparation le plus haut.

6. Procédé suivant la revendication 1, caractérisé en ce que l'intervalle entre étages de séparation est choisi de manière que l'étage de séparation qui se trouve juste au-dessus d'un étage se situe dans la zone d'injection de cet étage.

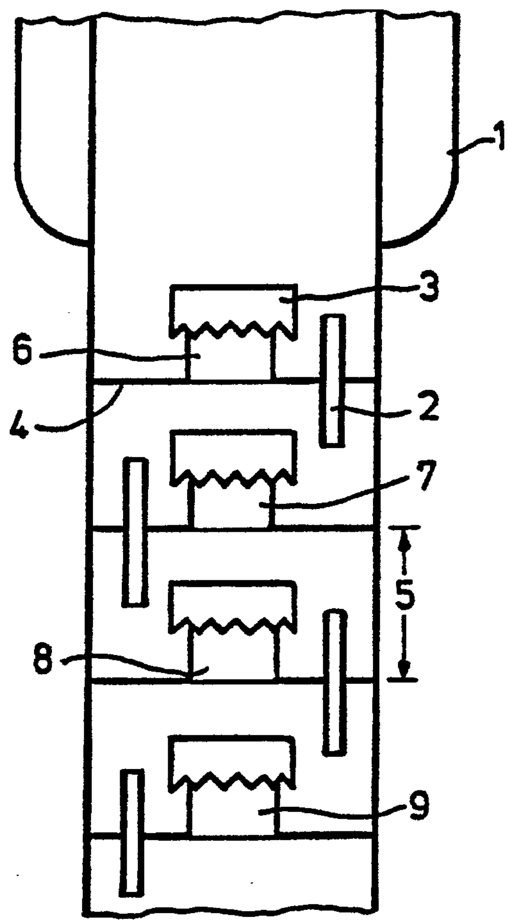


FIG. 1

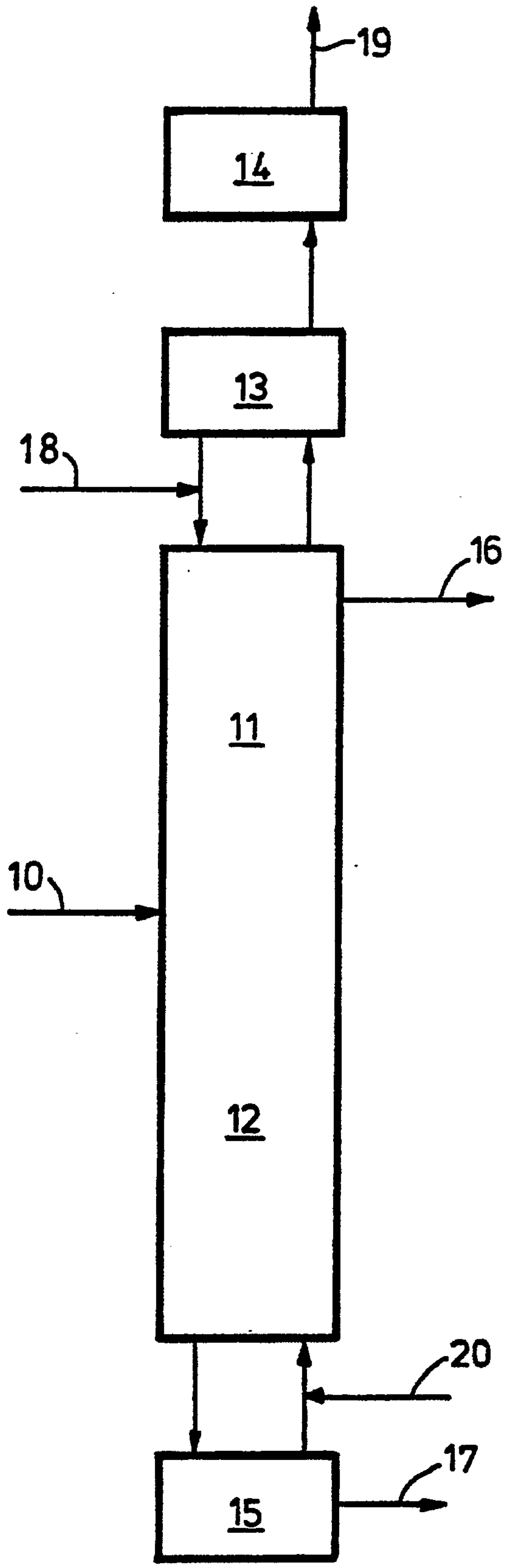


FIG. 2